(19) Europäisches Patentamt
European Patent Office
Office européen des brevets

(11) EP 2 236 173 B2

(12) # NOUVEAU FASCICULE DE BREVET EUROPEEN
Après la procédure d'opposition

(45) Date de publication et mention de la décision concernant l'opposition:
**25.11.2015 Bulletin 2015/48**

(45) Mention de la délivrance du brevet:
**22.08.2012 Bulletin 2012/34**

(21) Numéro de dépôt: **10150889.3**

(22) Date de dépôt: **15.01.2010**

(51) Int Cl.:
***A61Q 17/04*** *(2006.01)* ***A61K 8/34*** *(2006.01)*
***A61K 8/37*** *(2006.01)* ***A61K 8/88*** *(2006.01)*

(54) **Composition filtrante fluide anhydre oleoalcoolique comprenant un polycondensat polyamide lipophile**

Flüssige wasserfreie Öl-Alkohol-Filterzusammensetzung, die ein lipophiles Polyamid-Polykondensat enthält

Anhydrous oleoalcoholic liquid filtering composition including a lipophilic polyamide polycondensate

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **31.03.2009 FR 0952038**

(43) Date de publication de la demande:
**06.10.2010 Bulletin 2010/40**

(73) Titulaire: **L'Oréal**
**75008 Paris (FR)**

(72) Inventeurs:
- **Chevalier, Cyril**
  **91260, Juvisy-sur-Orge (FR)**
- **Gohier, Aurélia**
  **49000 Angers (FR)**
- **Guiramand, Carole**
  **78350, Jouy en Josas (FR)**

(74) Mandataire: **Miszputen, Laurent**
**L'Oréal**
**D.I.P.I.**
**25-29 Quai Aulagnier**
**92665 Asnières-sur-Seine Cedex (FR)**

(56) Documents cités:

| | |
|---|---|
| **EP-A- 1 813 266** | **WO-A-02/47620** |
| **FR-A- 2 918 563** | **US-A- 3 895 104** |
| **US-A- 4 731 242** | **US-A- 5 783 657** |
| **US-A1- 2005 197 479** | **US-B2- 6 592 857** |

- **L. D. RHEIN ET AL.: 'Surfactants in Personal Care Products and Decorative Cosmetics', vol. 135, 2007, CRC PRESS, NEW YORK pages 464 - 467**
- **Déclaration de James Richard Humphrey**

EP 2 236 173 B2

## Description

La présente invention concerne une composition anhydre fluide comprenant dans un milieu cosmétiquement acceptable :

a) au moins une huile hydrocarbonée **à des concentrations allant de 30 à 99,8% en poids par rapport au poids total de la composition** et
b) au moins un filtre UV organique lipophile et
c) au moins un mono-alcool linéaire en $C_1$-$C_3$ et
d) au moins un polycondensat polyamide lipophile.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

Dans le but d'assurer une protection de la peau et des matières kératiniques contre le rayonnement UV, on utilise généralement des compositions antisolaires comprenant des filtres organiques, actifs dans l'UV-A et actifs dans l'UV-B.

De nombreuses compositions cosmétiques destinées à la photoprotection (UV-A et/ou UV-B) de la peau ont été proposées à ce jour. On recherche tout particulièrement des formulations fluides qui assurent pour les utilisateurs une application facile sur la peau.

Parmi les compositions solaires fluides jusqu'alors proposées, les formulations anhydres type huile solaire sont particulièrement recherchées du fait de leur application facile et agréable sur la peau et de leur bonne résistance à l'eau. Cependant, elles sont peu répandues sur le marché des produits solaires du fait qu'il est difficile d'obtenir un facteur de protection solaire supérieur à 10 ainsi qu'un facteur de protection UVA répondant au ratio demandé par les du fait qu'il est difficile d'obtenir un facteur de protection solaire supérieur à 10 ainsi qu'un facteur de protection UVA répondant au ratio demandé par les différentes réglementations en matière de produits solaires, notamment supérieur à 5.

Le facteur de protection solaire (FPS) s'exprime mathématiquement par le rapport du temps d'irradiation nécessaire pour atteindre le seuil érythématogène avec le filtre UV au temps nécessaire pour atteindre le seuil érythématogène sans filtre UV. Il est évalué in vivo notamment selon la méthode Internationale publiée par Colipa / CTFA SA / JCIA (Mai 2006)

Pour caractériser la protection vis à vis des UV-A, la méthode PPD (Persistent Pigment Darkening), qui mesure la couleur de la peau observée 2 à 4 heures après une exposition de la peau aux UV-A, est particulièrement recommandée et utilisée. Cette méthode est adoptée depuis 1996 par la Japanese Cosmetic Industry Association (JCIA) en tant que procédure officielle de test pour l'étiquetage UV-A des produits et est fréquemment utilisée par les laboratoires de tests en Europe et aux Etats-Unis; (Japan Cosmetic Industry Association Technical Bulletin. Measurement Standards for UVA protection efficacy. Issued November 21, 1995 and efective of January 1, 1996).

Le facteur de protection $UVA_{PPD}$ (FP $UVA_{PPD}$) s'exprime mathématiquement par le rapport de la dose de rayonnement UV-A nécessaire pour atteindre le seuil de pigmentation avec le filtre UV (MPPDp) avec la dose de rayonnement UV-A nécessaire pour atteindre le seuil de pigmentation sans filtre UV (MPPDnp).

$$\text{FP UVA}_{PPD} = \frac{\text{MPPDp}}{\text{MPPDnp}}$$

En effet, les difficultés principales rencontrées dans la fabrication des huiles solaires pour essayer d'augmenter les indices de protection solaires sont d'obtenir à la fois une formule fluide, transparente et stable ayant une cosmétique

agréable.

On a déjà proposé dans les demandes EP1813266 et EP2014277 d'utiliser dans des compositions solaires fluides aqueuses du type émulsion des polycondensats polyamides lipophiles en particulier un polymère poly(ester-amide) à terminaison ester (ETPEA) ou un polymère polyamide à terminaison amide tertiaire (ATPA) afin d'obtenir des facteurs de protection solaire élevés.

Au cours de ses recherches, la demanderesse a constaté qu'en utilisant ce type de polycondensat polyamide lipophile dans une composition anhydre solaire fluide du type huile solaire, la formule ne restait pas fluide et devenait trop épaisse (formation d'un gel) et peu cosmétique. De plus, durant le stockage après 2 mois, la formule ainsi obtenue avait tendance à devenir hétérogène avec exudation d'huile : mélange de parties gélifiée et non gélifiée.

Il existe donc le besoin de trouver de nouvelles compositions solaires anhydres fluides, transparentes et stables dans le temps permettant d'atteindre des facteurs de protection solaire et UVA plus élevés.

Or, la Demanderesse vient maintenant de découvrir, de façon surprenante, que cet objectif pouvait être atteint avec une composition anhydre fluide comprenant dans un milieu cosmétiquement acceptable :

a) au moins une huile hydrocarbonée à des concentrations allant de 30 à 99,8% en poids par rapport au poids total de la composition et
b) au moins un filtre UV organique lipophile et
c) au moins un mono-alcool linéaire en $C_1$-$C_3$ et
d) au moins un polycondensat polyamide lipophile.

Cette découverte est à la base de la présente invention.

La présente invention concerne donc une composition anhydre fluide comprenant dans un milieu cosmétiquement acceptable :

a) au moins une huile hydrocarbonée **à des concentrations allant de 30 à 99,8% en poids par rapport au poids total de la composition** et
b) au moins un filtre UV organique lipophile et
c) au moins un mono-alcool linéaire en $C_1$-$C_3$ et
d) au moins un polycondensat polyamide lipophile.

D'autres caractéristiques, aspects et avantages de l'invention apparaîtront à la lecture de la description détaillée qui va suivre.

Par "cosmétiquement acceptable", on entend compatible avec la peau et/ou ses phanères, qui présente une couleur, une odeur et un toucher agréables et qui ne génère pas d'inconforts inacceptables (picotements, tiraillements, rougeurs), susceptibles de détourner la consommatrice d'utiliser cette composition.

Par « composition fluide », on entend au sens de l'invention une composition ne se présentant pas sous une forme solide et dont la viscosité mesurée à l'aide d'un viscosimètre Rhéomat 180 à 25°C à la vitesse de rotation de 200 RPM après 30 secondes de rotation est inférieure à 0,5 Pa.s et plus préférentiellement inférieure à 0,2 Pa.s et plus particulièrement allant de 0.0001 Pa.s à 0,1 Pa.s.

On entend par « composition anhydre » une composition contenant moins de 1 % en poids d'eau, voire moins de 0,5 % d'eau, et notamment exempte d'eau, l'eau n'étant pas ajoutée lors de la préparation de la composition mais correspondant à l'eau résiduelle apportée par les ingrédients mélangés.

Par "phase grasse liquide", au sens de la présente demande, on entend une phase grasse liquide à température ambiante (25°C) et pression atmosphérique (760mm de Hg), composée d'un ou plusieurs corps gras liquides à température ambiante, appelés aussi huiles, compatibles entre eux.

Par " lipophile" on entend tout composé cosmétique ou dermatologique susceptible d'être complètement dissous à l'état moléculaire dans une phase grasse liquide ou bien d'être solubilisé sous forme colloïdale (par exemple sous forme micellaire) dans une phase grasse liquide.

On entend par « huile » un corps gras liquide à la température ambiante (20 à 25°C).

On entend par "huile hydrocarbonée" toute huile comportant majoritairement des atomes de carbone et d'hydrogène, et éventuellement des groupements ester, éther, fluoré, acide carboxylique, alcool, silicone, amine, phényle et/ou aminoacide.

## POLYCONDENSAT POLYAMIDE LIPOPHILE

Par « polycondensat », on entend au sens de l'invention un polymère obtenu par polycondensation à savoir par réaction chimique entre des monomères possédant des groupes fonctionnels différents choisis en particulier parmi les fonctions acides, alcools et amines.

Par « polymère », on entend au sens de l'invention un composé ayant au moins 2 motifs de répétition, de préférence au moins 3 motifs de répétition et mieux encore 10 motifs de répétition.

Le ou les polycondensats polyamides lipophiles sont de préférence présents dans les compositions de l'invention dans des concentrations allant de 0,1 à 15% en poids par rapport au poids total de la composition, plus préférentiellement de 1 à 8 % en poids.

Les polycondensats polyamides lipophiles peuvent être notamment choisis parmi les polymères de polyamide comportant a) un squelette polymérique ayant des motifs de répétition hydrocarbonés pourvus d'au moins un motif amide non pendant, et éventuellement b) au moins une chaîne grasse pendante et/ou au moins une chaîne grasse terminale éventuellement fonctionnalisées, comprenant au moins 4 atomes de carbone et étant liées à ces motifs hydrocarbonés.

Par "chaînes fonctionnalisées" au sens de l'invention, on entend une chaîne alkyle comportant un ou plusieurs groupes fonctionnels ou réactifs notamment choisis parmi les groupes amides, hydroxyle, éther, oxyalkylène ou polyoxyalkylène, halogène, dont les groupes fluorés ou perfluorés, ester, siloxane, polysiloxane. En outre, les atomes d'hydrogène d'une ou plusieurs chaînes grasses peuvent être substitués au moins partiellement par des atomes de fluor.

Par "motifs de répétition hydrocarbonés", on entend au sens de l'invention un motif comportant de 2 à 80 atomes de carbone, et de préférence de 2 à 60 atomes de carbone, portant des atomes d'hydrogène et éventuellement des atomes d'oxygène, qui peut être linéaire, ramifié ou cyclique, saturé ou insaturé. Ces motifs comprennent, en outre, chacun au moins un groupement amide avantageusement non pendants et se trouvant dans le squelette polymérique.

Avantageusement, les chaînes pendantes sont liées directement à l'un au moins des atomes d'azote du squelette polymérique.

Le polycondensat polyamide lipophile peut comprendre entre les motifs hydrocarbonés des motifs siliconés ou des motifs oxyalkylénés.

En outre, le polycondensat polyamide lipophile de la composition de l'invention comprend avantageusement de 40 à 98 % de chaînes grasses par rapport au nombre total des motifs amide et des chaînes grasses et mieux de 50 à 95%.

De préférence, les chaînes grasses pendantes sont liées à l'un au moins des atomes d'azote des motifs amide du polymère. En particulier, les chaînes grasses de ce polyamide représentent de 40 à 98 % du nombre total des motifs amide et des chaînes grasses, et mieux de 50 à 95 %.

Avantageusement, le polycondensat polyamide lipophile présente une masse moléculaire moyenne en poids inférieure à 100 000 (notamment allant de 1000 à 100 000), en particulier inférieure à 50 000 (notamment allant de 1000 à 50 000), et plus particulièrement allant de 1000 à 30 000, de préférence de 2000 à 20 000, et mieux de 2000 à 10 000.

Le polycondensat polyamide lipophile est non soluble dans l'eau, notamment à 25 °C. En particulier, il ne comporte pas de groupe ionique.

Comme polycondensats polyamides lipophiles préférés utilisables dans l'invention, on peut citer les polyamides ramifiés par des chaînes grasses pendantes et/ou des chaînes grasses terminales ayant de 6 à 120 atomes de carbone et mieux de 8 à 120 et notamment de 12 à 68 atomes de carbone, chaque chaîne grasse terminale étant liée au squelette polyamide par au moins un groupe de liaison L. Le groupe de liaison L peut être choisi parmi les groupes ester, éther, amine, urée, uréthane, thioester, thioéther, thiurée, thiouréthane. De préférence, ces polymères comportent une chaîne grasse à chaque extrémité du squelette polyamide.

Ces polymères sont de préférence des polymères résultant d'une polycondensation entre un diacide carboxylique ayant au moins 32 atomes de carbone (ayant notamment de 32 à 44 atomes de carbone) avec une amine choisie parmi les diamines ayant au moins 2 atomes de carbone (notamment de 2 à 36 atomes de carbone) et les triamines ayant au moins 2 atomes de carbone (notamment de 2 à 36 atomes de carbone). Le diacide est de préférence un dimère issu d'acide gras à insaturation éthylénique ayant au moins 16 atomes de carbone, de préférence de 16 à 24 atomes de carbone, comme l'acide oléique, linoléique ou linolénique. La diamine est de préférence l'éthylène diamine, l'hexylène diamine, l'hexaméthylène diamine. La triamine est par exemple l'éthylène triamine. Pour les polymères comportant un ou 2 groupements d'acide carboxylique terminaux, il est avantageux de les estérifier par un monoalcool ayant au moins 4 atomes de carbone, de préférence de 10 à 36 atomes de carbone et mieux de 12 à 24 et encore mieux de 16 à 24, par exemple 18 atomes de carbone.

Ces polymères sont plus spécialement ceux décrits dans le document US-A-5783657 de la société Union Camp.

Chacun de ces polymères satisfait notamment à la formule (B) suivante :

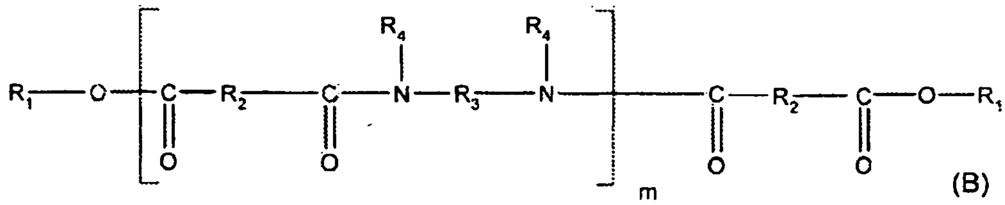

(B)

dans laquelle :

- m désigne un nombre entier de motifs amide tel que le nombre de groupes ester représente de 10 % à 50 % du nombre total des groupes ester et amide;
- $R_1$ est à chaque occurrence indépendamment un groupe alkyle ou alcényle ayant au moins 4 atomes de carbone et notamment de 4 à 24 atomes de carbone ;
- $R_2$ représente à chaque occurrence indépendamment un groupe hydrocarboné en $C_4$ à $C_{42}$ à condition que 50 % des groupes $R_2$ représentent un groupe hydrocarboné en C30 à C42 ;
- $R_3$ représente à chaque occurrence indépendamment un groupe organique pourvu d'au moins 2 atomes de carbone, d'atomes d'hydrogène et optionnellement d'un ou plusieurs atomes d'oxygène ou d'azote ;
- et $R_4$ représente à chaque occurrence indépendamment un atome d'hydrogène, un groupe alkyle en $C_1$ à $C_{10}$ ou une liaison directe à $R_3$ ou à un autre $R_4$ de sorte que l'atome d'azote auquel sont liés à la fois $R_3$ et $R_4$ fasse partie d'une structure hétérocyclique définie par $R_4$-N-$R_3$, avec au moins 50 % des $R_4$ représentant un atome d'hydrogène.

Dans le cas particulier de la formule (B), les chaînes grasses terminales éventuellement fonctionnalisées au sens de l'invention sont des chaînes terminales liées au dernier atome d'azote, du squelette polyamide.

En particulier, les groupes ester de la formule (B), qui font partie des chaînes grasses terminales et/ou pendantes au sens de l'invention, représentent de 15 à 40 % du nombre total des groupes ester et amide et mieux de 20 à 35 %.

De plus, m représente avantageusement un nombre entier allant de 1 à 5 et mieux supérieur à 2.

De préférence, $R_1$ est un groupe alkyle en $C_{12}$ à $C_{22}$ et de préférence en $C_{16}$ à $C_{22}$. Avantageusement, $R_2$ peut être un groupe hydrocarboné (alkylène) en $C_{10}$ à $C_{42}$. De préférence, 50 % au moins et mieux au moins 75 % des $R_2$ sont des groupes ayant de 30 à 42 atomes de carbone. Les autres $R_2$ sont des groupes hydrogénés en C4 à C19 et même en $C_4$ à $C_{12}$.

De préférence, $R_3$ représente un groupe hydrocarboné en $C_2$ à $C_{36}$ ou un groupe polyoxyalkyléné et $R_4$ représente un atome d'hydrogène. De préférence, $R_3$ représente un groupe hydrocarboné en $C_2$ à $C_{12}$.

Les groupes hydrocarbonés peuvent être des groupes linéaires, cycliques ou ramifiés, saturés ou insaturés. Par ailleurs, les groupes alkyle et alkylène peuvent être des groupes linéaires ou ramifiés, saturés ou non.

En général, les polymères de formule (B) se présentent sous forme de mélanges de polymères, ces mélanges pouvant en outre contenir un produit de synthèse correspondant à un composé de formule (B) où m vaut 0, c'est-à-dire un diester.

Selon une forme particulièrement préférée de l'invention, on utilisera un mélange de copolymères d'un diacide en $C_{36}$ condensé sur l'éthylène diamine ; les groupes ester terminaux résultent de l'estérification des terminaisons d'acide restantes par l'alcool cétylique, stéarylique ou leurs mélanges (appelés aussi alcool cétylstéarylique) (nom INCI: ETHYLENEDIAMINE/STEARYL DIMER DILINOLEATE COPOLYMER). Sa masse moléculaire moyenne en poids est de préférence de 6000. Ces mélanges sont notamment vendus par la société ARIZONA CHEMICAL sous les noms commerciaux UNICLEAR 80 et UNICLEAR 100 VG respectivement sous forme de gel à 80 % (en matière active) dans une huile minérale et à 100 % (en matière active). Ils ont un point de ramollissement de 88°C à 94°C.

Selon une forme particulièrement préférée de l'invention, on utilisera plus particulièrement un copolymère de diacide linoléique hydrogéné, d'éthylènediamine, de di($C_{14}$-$C_{18}$)alkylamine(s) (nom INCI : ETHYLENEDIAMIDE/HYDROGENATED DIMER DILINOLEATE COPOLYMER BIS-DI-$C_{14}$-$C_{18}$ ALKYL AMIDE). Ce copolymère est notamment vendu sous le nom commercial SYLVACLEAR A200V par la société ARIZONA CHEMICAL.

Selon une forme particulièrement préférée de l'invention, on utilisera plus particulièrement un copolymère de diacide linoléique hydrogéné, d'éthylènediamine, de néopentylglycol et d'alcool stéarylique (nom INCI : BIS-STEARYL ETHYLENEDIAMINE/NEOPENTYL GLYCOL/STEARYL HYDROGENATED DIMER DILINOLEATE COPOLYMER). Ce copolymère est notamment vendu sous le nom commercial SYLVACLEAR C75 V par la société ARIZONA CHEMICAL.

Comme polycondensats polyamides utilisables dans l'invention, on peut encore citer ceux comprenant au moins une chaîne grasse terminale liée au squelette polymérique par au moins un groupe de liaison éther ou polyéther (il est dit alors éther terminated poly(éther)amide). De tels polymères sont décrits par exemple dans le document US 6 399 713.

Les polyamides conformes à l'invention ont avantageusement une température de ramollissement supérieure

à 65°C et pouvant aller jusqu'à 190°C. De préférence, il présente une température de ramollissement allant de 70 à 130°C et mieux de 80 à 105°C. Le polyamide est en particulier un polymère non cireux.

Comme polycondensats polyamides utilisables dans l'invention, on peut aussi citer les résines polyamides résultant de la condensation d'un acide di-carboxylique aliphatique et d'une diamine (incluant les composés ayant plus de 2 groupes carbonyle et 2 groupes amine), les groupes carbonyle et amine de motifs unitaires adjacents étant condensés par une liaison amide. Ces résines polyamides sont notamment celles commercialisées sous la marque Versamid® par les sociétés General Mills, Inc. et Henkel Corp. (Versamid 930, 744 ou 1655) ou par la société Olin Mathieson Chemical Corp., sous la marque Onamid® notamment Onamid S ou C. Ces résines ont une masse moléculaire moyenne en poids allant de 6000 à 9000. Pour plus d'information sur ces polyamides, on peut se référer aux documents US 3645705 et US 3148125. Plus spécialement, on utilise les Versamid® 930 ou 744.

On peut aussi utiliser les polyamides vendus par la société Arizona Chemical sous les références Uni-Rez (2658, 2931, 2970, 2621, 2613, 2624, 2665, 1554, 2623, 2662) et le produit vendu sous la référence Macromelt 6212 par la société Henkel. Pour plus d'information sur ces polyamides, on peut se référer au document US 5500209.

Les compositions de l'invention comprennent au moins une huile hydrocarbonée pouvant être volatile ou non-volatile.

Par " huile volatile", on entend au sens de l'invention une huile susceptible de s'évaporer au contact de la peau ou de la fibre kératinique en moins d'une heure, à température ambiante et pression atmosphérique. Le ou les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

Par "huile non volatile", on entend une huile restant sur la peau ou la fibre kératinique à température ambiante et pression atmosphérique au moins plusieurs heures et ayant notamment une pression de vapeur inférieure à $10^{-3}$ mm de Hg (0,13 Pa).

Le ou les huiles hydrocarbonées sont présentes dans la composition de l'invention à des concentrations allant de 30 à 99,8% en poids et plus préférentiellement de 40 à 90% en poids par rapport au poids total de la composition.

Comme huiles hydrocarbonées non volatiles utilisables selon l'invention, on peut notamment citer :

1. (i) les huiles hydrocarbonées d'origine végétale telles que les triesters de glycérides qui sont en général des triesters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,
2. (ii) les éthers de synthèse ayant de 10 à 40 atomes de carbone ;
3. (iii) les hydrocarbures linéaires ou ramifiés, d'origine minérale ou synthétique tels que la vaseline, les polydécènes, le polyisobutène hydrogéné tel que le parléam, le squalane, et leurs mélanges ;
4. (iv) les esters de synthèse comme les huiles de formule RCOOR' dans laquelle R représente le reste d'un acide gras linéaire ou ramifié comportant de 1 à 40 atomes de carbone et R' représente une chaîne hydrocarbonée notamment ramifiée contenant de 1 à 40 atomes de carbone à condition que R + R' soit ≥ 10, comme par exemple l'huile de Purcellin (octanoate de cétostéaryle), le myristate d'isopropyle, le palmitate d'isopropyle, le benzoate d'alcools en $C_{12}$-$C_{15}$ comme le produit vendu sous la dénomination commerciale « FINSOLV TN » ou « WITCONOL TN » par la société WITCO ou « TEGOSOFT TN » par la société EVONIK GOLDSCHMIDT , le Benzoate de 2-éthylphenyle comme le produit commercial vendu sous le nom « X-TEND 226» par la société ISP, le lanolate d'isopropyle, le laurate d'hexyle, l'adipate de diisopropyle, l'isononanoate d'isononyle, l'érucate d'oléyle, le palmitate de 2-éthyl-hexyle, l'isostéarate d'isostéaryle, des octanoates, décanoates ou ricinoléates d'alcools ou de polyalcools comme le dioctanoate de propylène glycol ; les esters hydroxylés comme le lactate d'isostéaryle, le malate de diisostéaryle ; et les esters du pentaérythritol ; les citrates ou tartrates comme les tartrates de di-alkyle linéaire en $C_{12}$-$C_{13}$ tels que ceux vendus sous le nom COSMACOL ETI par la Société ENICHEM AUGUSTA INDUSTRIALE ainsi que les tartrates de di-alkyle linéaire en $C_{14}$-$C_{15}$ tels que ceux vendus sous le nom COSMACOL ETL par la même société ; les acétates.
5. (v) les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
6. (vi) les acides gras supérieurs tels que l'acide oléique, l'acide linoléique, l'acide linolénique ;

7. (vii) les carbonates comme le dicaprylyl carbonate comme le produit vendu sous la dénomination « CETIOL CC » par la société COGNIS ;

8. (viii) les amides grasses comme l'Isopropyl N-lauroyl sarcosinate comme le produit vendu sous le nom commercial ELDEW SL205" de chez AJINOMOTO et leurs mélanges.

Les huiles hydrocarbonées volatiles peuvent être choisies parmi les huiles hydrocarbonées ayant de 8 à 16 atomes de carbones, et notamment les alcanes ramifiés en $C_8$-$C_{16}$ comme les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, les huiles vendues sous les noms commerciaux d'Isopars ou de Permetyls, les esters ramifiés en $C_8$-$C_{16}$ le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. Selon un mode de réalisation, le solvant volatil est choisi parmi les huiles volatiles hydrocarbonées ayant de 8 à 16 atomes de carbone et leurs mélanges.

On peut citer aussi les alcanes décrits dans les demandes de brevets de la société Cognis WO 2007/068371, ou WO2008/155059 (mélanges d'alcanes distincts et différant d'au moins un carbone). Ces alcanes sont obtenus à partir d'alcools gras, eux-mêmes obtenus à partir d'huile de coprah ou de palme. on peut citer les mélanges de n-undécane ($C_{11}$) et de n-tridécane ($C_{13}$) obtenus aux exemples 1 et 2 de la demande WO2008/155059 de la Société Cognis. On peut également citer le n-dodécane ($C_{12}$) et le n-tétradécane ($C_{14}$) vendus par Sasol respectivement sous les références PARAFOL 12-97 et PARAFOL 14-97, ainsi que leurs mélanges.

Parmi les huiles hydrocarbonées utilisables selon l'invention, on préférera plus particulièrement les triesters de glycéride et notamment les triglycérides des acides caprylique/caprique , les esters de synthèse et notamment l'isononanoate d'isononyle, l'érucate d'oléyle, le benzoate d'alcools en $C_{12}$-$C_{15}$ et les alcools gras notamment l'octyldodécanol.

## <u>HUILES ADDITIONNELLES</u>

Les compositions selon l'invention peuvent contenir en plus une ou plusieurs huiles additionnelles qui seront choisies de préférence parmi les huiles de silicone volatiles, les huiles de silicone non volatiles.

La ou les huiles siliconées sont présentes dans la phase grasse liquide à des concentrations allant de 0,1 à 20% en poids et de préférence de 0,1 à 5% en poids par rapport au poids total de la phase grasse liquide.

Les huiles siliconées non volatiles peuvent être choisies notamment parmi les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Comme huiles volatiles siliconées, on peut citer par exemple les huiles de silicones linéaires ou cycliques volatiles, notamment celles ayant une viscosité $\leq$ 8 centistokes (8 $10^{-6}$ $m^2/s$), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

On peut également citer les huiles linéaires alkyltrisiloxanes volatiles de formule générale (I) :

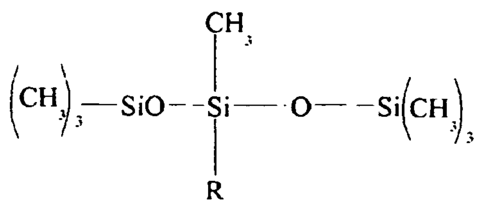

où R représente un groupe alkyle comprenant de 2 à 4 atomes de carbone et dont un ou plusieurs atomes d'hydrogène peuvent être substitués par un atome de fluor ou de chlore.

Parmi les huiles de formule générale (I), on peut citer :

le 3-butyl 1,1,1,3,5,5,5-heptaméthyl trisiloxane,

le 3-propyl 1,1,1,3,5,5,5-heptaméthyl trisiloxane, et

le 3-éthyl 1,1,1,3,5,5,5-heptaméthyl trisiloxane,

correspondant aux huiles de formule (I) pour lesquelles R est respectivement un groupe butyle, un groupe propyle ou un groupe éthyle.

**MONOALCOOL EN $C_1$-$C_3$ LINEAIRE**

Le ou les mono-alcools en $C_1$-$C_3$ présents dans les compositions de l'invention peuvent être choisis parmi le méthanol, l'éthanol, le propanol ou leurs mélanges. On choisira plus particulièrement l'éthanol.

Ils sont en général présents à des concentrations allant de 0,1 à 40% en poids, plus préférentiellement de 2 à 10 % en poids par rapport au poids total de la composition.

**FILTRES UV ORGANIQUES LIPOPHILES**

Ils peuvent notamment être choisis parmi les dérivés de l'acide paraaminobenzoique, les dérivés salicyliques, les dérivés cinnamiques, les benzophénones et aminobenzophénones, les dérivés anthraniliques, les dérivés du dibenzoylméthane, les dérivés de β,β-diphénylacrylate, les dérivés du benzylidène camphre, les dérivés du phényl benzimidazole, les dérivés de benzotriazole, les dérivés triazine, les bis-résorcinyl triazines, les dérivés d'imidazolines, les dérivés du benzalmalonate, les dérivés de 4,4-diarylbutadiène, les dérivés de benzoxazole, les mérocyanines et leurs mélanges.

Parmi les filtres UVA organiques lipophiles capables d'absorber les UV de 320 à 400 nm, on peut citer

**Les dérivés du dibenzoylméthane :**

le 4-isopropyl-dibenzoylméthane, vendu sous la dénomination de "EUSOLEX 8020" par la Société MERCK, et répondant à la formule suivante :

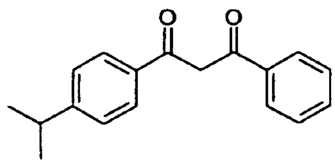

- le 1-(4-methoxy-1-benzofuran-5-yl)-3-phenylpropane-1,3-dione, proposé à la vente par la société QUEST sous le nom de Pongamol de formule :

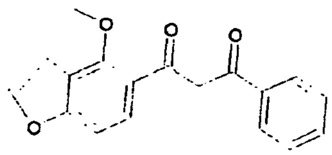

- le 1-(4-tert-butylphenyl)-3-(2-hydroxyphenyl)propane-1,3-dione de formule :

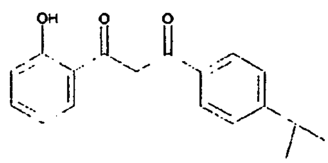

- le Butyl Methoxydibenzoylmethane vendu notamment sous le nom commercial « PARSOL 1789 » par HOFFMANN LA ROCHE,

**Les aminobenzophénones**

2-(4-diethylamino-2-hydroxybenzoyl)-benzoate de n-hexyle vendu sous le nom commercial« UVINUL A +»

**Les dérivés anthraniliques** :

Menthyl anthranilate vendu sous le nom commercial « NEO HELIOPAN MA » par HAARMANN et REIMER,

**Les dérivés de 4,4-diarylbutadiène** :

1,1-dicarboxy (2,2'-diméthyl-propyl)-4,4-diphénylbutadiène

**Les préférentiels sont :**

Butyl Methoxydibenzoylmethane
2-(4-diethylamino-2-hydroxybenzoyl)-benzoate de n-hexyle

Parmi les filtres UVB organiques lipophiles capables d'absorber les UV de 280 à 320 nm, on peut citer

**Les para-aminobenzoates** :

Ethyl PABA
Ethyl Dihydroxypropyl PABA
Ethylhexyl Dimethyl PABA (ESCALOL 507 de ISP)

**Les dérivés salicyliques** :

Homosalate vendu sous le nom « Eusolex HMS » par Rona/EM Industries,

- Ethylhexyl Salicylate vendu sous le nom « NEO HELIOPAN OS » par HAARMANN et REIMER,
- Dipropyleneglycol Salicylate vendu sous le nom « DIPSAL » par SCHER, TEA Salicylate, vendu sous le nom « NEO HELIOPAN TS » par Haarmann et REIMER,

**Les cinnamates**

Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
Diisopropyl Methylcinnamate,
Cinoxate,
Glyceryl Ethylhexanoate Diméthoxycinnamate

**Les dérivés de β,β'-diphénylacrylate :**

Octocrylène, vendu notamment sous le nom commercial « UVINUL N539 » par BASF,
Etocrylène, vendu notamment sous le nom commercial « UVINUL N35 » par BASF,

**Les dérivés du benzylidène camphre :**

3-Benzylidène camphre fabriqué sous le nom « MEXORYL SD » par CHIMEX, Méthylbenzylidène camphre vendu sous le nom « EUSOLEX 6300 » par MERCK,
Polyacrylamidométhyle Benzylidène Camphre fabriqué sous le nom « MEXORYL SW » par CHIMEX,

**Les dérivés de triazine :**

Ethylhexyl triazone vendu notamment sous le nom commercial « UVINUL T150 » par BASF,
2,4,6-tris(4'-amino benzalmalonate de dinéopentyle)-s-triazine
la 2,4,6-tris-(4'-amino benzalmalonate de diisobutyle)-s- triazine,

2,4-bis(4'-amino benzalmalonate de dinéopentyle)-6-(4'-aminobenzoate de n-butyle)-s-triazine,
2,4-bis(4'-amino benzoate de n-butyle)-6-(aminopropyltrisiloxane)-s-triazine,

**<u>Les dérivés d'imidazolines :</u>**

Ethylhexyl Dimethoxybenzylidene Dioxoimidazoline Propionate,

**<u>Les dérivés du benzalmalonate :</u>**

Polyorganosiloxanes à fonction benzalmalonate tels que le Polysilicone-15 vendu sous la dénomination commerciale « PARSOL SLX » par HOFFMANN LA ROCHE
Di-neopentyl 4'-méthoxybenzalmalonate,

**<u>Les dérivés de mérocyanine</u>**

Octyl-5-N,N-diethylamino-2-phenysulfonyl-2,4-pentadienoate

**Les préférentiels sont :**

Homosalate
Ethylhexyl Salicylate
Ethylhexyl Methoxycinnamate
Octocrylène
Ethylhexyl triazone
2,4-bis(4'-amino benzoate de n-butyle)-6-(aminopropyltrisiloxane)-s-triazine, Octyl-5-N,N-diethylamino-2-phenysulfonyl-2,4-pentadienoate

Parmi les filtres organiques lipophiles à spectre large capables d'absorber les UVA et les UVB, on peut citer

**<u>Les dérivés de benzophénone</u>**

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-5,
Benzophenone-6 vendu sous le nom commercial « Helisorb 11 » par Norquay
Benzophenone-8 vendu sous le nom commercial « Spectra-Sorb UV-24 » par American Cyanamid
Benzophenone-10,
Benzophenone-11,
Benzophenone-12,

**<u>Dérivés de benzotriazole :</u>**

Drometrizole Trisiloxane vendu sous le nom « Silatrizole » par RHODIA CHIMIE Bumetrizole vendu sous le nom TINOGUARD AS par CIBA-GEIGY

**<u>Les dérivés bis-résorcinyl triazines</u>**

Bis-Ethylhexyloxyphénol Méthoxyphenyl Triazine vendu sous le nom commercial « TINOSORB S » par CIBA GEIGY,

**<u>Les dérivés de benzoxazole :</u>**

2,4-bis-[5-1 (diméthylpropyl)benzoxazol-2-yl-(4-phenyl)-imino]-6-(2-ethylhexyl)-imino-1,3,5-triazine vendu sous le nom d'Uvasorb K2A par Sigma 3V

**Les préférentiels sont :**

Benzophenone-3

Drometrizole Trisiloxane
Bis-Ethylhexyloxyphénol Méthoxyphenyl Triazine
Les filtres organiques lipophiles sont généralement présents dans les compositions selon l'invention dans des proportions allant de 0,1 à 50% en poids par rapport au poids total de la composition, et de préférence allant de 2 à 30% en poids par rapport au poids total de la composition.

Selon une forme particulièrement préférée de l'invention, les compositions seront transparentes et présenteront de préférence une turbidité inférieure à 1000 NTU (Unités de Turbidité Nephélométique) à 25°C, de préférence inférieure à 50 NTU à 25°C et encore plus préférentiellement inférieure à 15 NTU, mesurée avec un appareil 2100P Turbidimeter de la société HACH.

Selon une forme particulièrement préférée de l'invention, les compositions présenteront un FPS supérieur à 10, voire supérieur 15 et même supérieur à 20.

Selon une forme particulièrement préférée de l'invention, les compositions présenteront un FP $UVA_{PPD}$ supérieur à 5, elles respectent aussi la réglementation notamment européenne qui veut que le ratio SPF /PPD soit inférieur à 3.

**ADDITIFS**

La composition huileuse du produit de l'invention peut également contenir différents additifs qui peuvent être solubles dans la phase huileuse, ou être en dispersion dans ladite phase huileuse notamment choisis parmi les colorants lipophiles, les actifs lipophiles, les polymères lipophiles autres que les polycondensats polyamides de l'invention, les solvants organiques, les conservateurs, les agents insectifuges, les huiles essentielles, les parfums, les émollients, les propulseurs.

Parmi les actifs cosmétiques lipophiles, on peut citer par exemple les antioxydants, les agents kératolytiques tels que les acides N-alkyl salicyliques, par exemple l'acide N-octanoyl-5 salicylique ; les vitamines comme la vitamine E (tocophérol et dérivés), la vitamine A (rétinol et dérivés) ; les adoucissants et tout actif lipophile habituellement utilisé dans le soin de la peau ou des cheveux.

Comme polymères lipophiles additionnels, on peut citer les copolymères blocs dérivés du styrène comme le copolymère styrène/ethylene-butylene/styrene tel que le produit commercialisé sous la dénomination Kraton G-1650E par la société Kraton Polymers. ; les copolymères d'acide acrylique ou méthacrylique, tels que le copolymère Acrylate/stearyl acrylate/dimethicone methacrylate commercialisé sous la dénomination KP 561 P par la société Shin Etsu ; les Poly-C10-30-Alkyl Acrylates comme le produit commercialisé sous la dénomination Interlimer IPA 13-1 par la société Landec

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Un autre objet de la présente invention est constitué par l'utilisation des compositions selon l'invention telles que ci-dessus définies pour la fabrication de produits pour le traitement cosmétique de la peau, des ongles, des cheveux, des cils, sourcils et/ou du cuir chevelu, notamment des produits de soin, des produits de protection solaire.

Les compositions cosmétiques selon l'invention peuvent par exemple être utilisées comme produit de soin et/ou de protection solaire et /ou de photoprotection quotidienne et/ou de maquillage, et/ou de capillaire pour le visage et/ou le corps et/ou les cheveux de consistance liquide.

**PARFUMS**

Selon une forme particulière de l'invention, les compositions cosmétiques selon l'invention peuvent constituer des produits parfumants et contenir en plus une substance parfumante.

Par produit parfumant, on entend toute composition laissant après application sur les matières kétatiniques un parfum.

Par « substance parfumante», on entend tout parfum ou arôme susceptible de parfumer la peau et les matières kératiniques humaines en général comprenant la peau, les cheveux, le cuir chevelu, les lèvres, les ongles.

Comme substance parfumante, on peut utiliser dans la composition de l'invention, les parfums et les arômes d'origine naturelle ou synthétique et leurs mélanges. Comme parfums et arômes d'origine naturelle, on peut citer par exemple les extraits de fleurs (lis, lavande, rose, jasmin, ilang-ilang), de tiges et de feuilles (patchouli, géranium, petit-grain), de fruits (coriandre, anis, cumin, genièvre), d'écorces de fruits (bergamote, citron, orange), de racines (angélique, céleri, cardamome, iris, acore), de bois (bois de pin, santal, gaïac, cèdre rose), d'herbes et de graminées (estragon, lemon grass, sauge, thym), d'aiguilles et de branches (épicéa, sapin, pin, pin nain), de résines et de baumes (galbanum, élémi, benjoin, myrrhe, oliban, opopanax).

Comme substance parfumante d'origine synthétique, on peut citer par exemple les composés du type ester, éther, aldéhyde, cétone, alcool aromatique et hydrocarbure.

Comme esters, on peut citer en particulier l'acétate de benzyle, le benzoate de benzyle, l'isobutyrate de phénoxyéthyle, l'acétate de p-tert-butylcyclohexyle, l'acétate de citronellyle, le formiate de citronellyle, l'acétate de géranyle, l'acétate de linalyle, l'acétate de diméthyl-benzylcarbinyle, l'acétate de phényléthyle, le benzoate de linalyle, le formiate de benzyle, le glycinate d'éthylméthylphényle, le propionate d'alkylcyclohexyle, le propionate de styralyle et le salicylate de benzyle.

Comme éthers, on peut citer le benzyléthyléther.

Comme aldéhydes, on peut citer par exemple les alcanals linéaires comportant de 8 à 18 atomes de carbone, le citral, le citronellal, le citronellytoxyacétaldéhyde, le cyclamènaldéhyde, l'hydroxycitronellal, le lilial et le bourgeonal.

Comme cétones, on peut citer par exemple les ionones comme l'alpha-isométhylionone, et la méthylcédrylcétone.

Parmi les alcool aromatiques et notamment terpéniques, on peut citer l'anéthol, le citronellol, l'eugénol, l'isoeugénol, le géraniol, le linalol, le phényléthylalcool et le terpinéol.

Comme hydrocarbures, on peut citer notamment les terpènes. Ces composés se présentent souvent sous forme de mélange de deux ou plus de ces substances odorantes.

Par ailleurs, on peut aussi utiliser des huiles essentielles, composants d'arômes, comme par exemple les essences de sauge, de camomille, de girofle, de mélisse, de menthe, de feuilles de cannelier, de fleurs de tilleul, de genièvre, de vétiver, d'olibian, de galbanum, de labolanum et de lavandin.

On peut utiliser comme substance parfumante, seule ou en mélange, l'essence de bergamote, le dihydromyrcénol, le lilial, le lyral, le citronellol, l'alcool phényléthylique, l'alpha-hexylcinnamaldéhyde, le géraniol, la benzylacétone, le cyclamènaldéhyde, le linalol, l'ambroxane, l'indol, l'hédione, la sandelice, les essences de citron, de mandarine et d'orange, le glycolate d'allylamine, le cyclovertal, l'essence de lavandin, l'essence de sauge, le bétadamascone, l'essence de géranium, le salicylate de cyclohexyle, l'acide phénylacétique, l'acétate de géranyle, l'acétate de benzyle, l'oxyde de rose.

On peut aussi utiliser un mélange de différentes substances parfumantes qui engendrent en commun une note plaisante pour l'utilisateur. Parmi les notes olfactives connues, on peut citer par exemple les parfums hespéridés, les aromatiques, les parfums floraux, les musqués, les parfums fruités, les épicés, les parfums orientaux, les parfums marins, les notes aquatiques, les parfums chyprés, les parfums boisés, les fougères et leurs mélanges.

La quantité de substance(s) parfumante(s) sera de préférence de 5 à 25 % en poids, mieux de 10 à 20% en poids par rapport au poids total de la composition.

## AGENTS COLORANTS ADDITIONNELS

Selon une autre forme particulière de l'invention, les compositions de l'invention peuvent comporter en plus un ou plusieurs agents de coloration additionnels.

Les agents de coloration additionnels peuvent être également choisis parmi les colorants directs synthétiques ou naturels. Il peut s'agir de colorants organiques ou minéraux.

Les colorants organiques liposolubles, synthétiques ou naturels sont, par exemple, le DC Red 17, le DC Red 21, le DC Red 27, le DC Green 6, le DC

Yellow 11, le DC Violet 2, le DC Orange 5, le rouge Soudan, les carotènes (le β-carotène, le lycopène), les xanthophylles (capsanthine, capsorubine, lutéine), l'huile de palme, le brun Soudan, le jaune quinoléine, le rocou, le curcumin.

Les agents de coloration additionnels peuvent être également choisis parmi les matières colorantes particulaires qui sont choisies de préférence parmi les pigments, les nacres ou pigments interférentiels, les paillettes.

Par pigments, il faut comprendre des particules de toute forme, blanches ou colorées, minérales ou organiques, insolubles dans le milieu physiologique, destinées à colorer la composition.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

On peut également citer les pigments à effet tels les particules comportant un substrat organique ou minéral, naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, l'oxyde de chrome et leurs mélanges.

Au sens de la présente invention, l'expression "particules interférentielles ou nacres" désigne toute particule possédant généralement une structure multicouche telle qu'elle permette la création d'un effet de couleur par interférence

des rayons lumineux qui diffractent et diffusent différemment selon la nature des couches. Les effets colorants obtenus sont liés à la structure lamellaire de ces particules et dérivent des lois physiques de l'optique des couches minces (voir : Pearl Lustre Pigments - Physical principles, properties, applications - R. Maisch, M. Weigand. Verlag Moderne Industrie). Ainsi, ces particules peuvent présenter des couleurs variant selon l'angle d'observation et l'incidence de la lumière.

Au sens de la présente invention, une structure multicouche entend désigner indifféremment une structure formée d'un substrat recouvert d'une unique couche ou une structure formée d'un substrat recouvert d'au moins deux voire de plusieurs couches consécutives.

La structure multicouche peut ainsi comporter une voire au moins deux couches, chaque couche indépendamment ou non de la (ou les) autre(s) couche(s) étant réalisée(s) en au moins un matériau choisi dans le groupe constitué par les matériaux suivants : $MgF_2$, $CeF_3$, ZnS, ZnSe, Si, $SiO_2$, Ge, Te, $Fe_2O_3$, Pt, Va, $Al2O_3$, MgO, $Y2O_3$, $S_2O_3$, SiO, $HfO_2$, $ZrO_2$, $CeO_2$, $Nb2O_5$, $Ta_2O_5$, TiO2, Ag, Al, Au, Cu, Rb, Ti, Ta, W, Zn, MoS2, cryolithe, alliages, polymères et leurs associations.

Généralement, la structure multicouche est de nature inorganique.

Plus particulièrement, les particules interférentielles considérées selon l'invention peuvent être des pigments interférentiels, ou encore des nacres naturelles ou synthétiques, monocouches ou multicouches, en particulier formées d'un substrat naturel à base, entre autres, de mica et qui est recouvert d'une ou plusieurs couches d'oxyde métallique.

Les particules interférentielles selon l'invention sont caractérisées en ce que 50% de la population massique a un diamètre (d50) inférieur à 40 $\mu$m, plus particulièrement inférieur à 30 $\mu$m, notamment inférieur à 20 $\mu$m, et en particulier inférieur à 15 $\mu$m, mesurée par un granulomètre laser, tel que par exemple le Mastersizer 2000® de Malvernet ou le BI90 +® de Broockhaven Instrument Corporation.

Conviennent tout particulièrement à l'invention, les nacres de type mica/oxyde d'étain/oxyde de titane telles que par exemple celles commercialisées sous les dénominations TIMIRON SILK BLUE®, TIMIRON SILK RED®, TIMIRON SILK GREEN®, TIMIRON SILK GOLD® et TIMIRON SUPER SILK® proposées par la société MERCK et les nacres mica/oxyde de fer/oxyde de titane telles que par exemple les FLAMENCO SATIN BLUE®, FLAMENCO SATIN RED® et FLAMENCO SATIN VIOLET® et FLAMENCO ORANGE 320C proposées par la société ENGELHARD et leurs mélanges.

Plus précisément, ces pigments peuvent être présents dans des quantités allant de 0,01 à 10% en poids et de préférence allant de 0,1 à 5% en poids par rapport au poids total de la composition.

**<u>COMPOSITIONS VAPORISABLES</u>**

Les compositions selon l'invention peuvent se présenter sous forme d'huile vaporisable appliquée sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517.

Ces compositions peuvent être aussi imprégnées sur des supports type lingettes, ou elles peuvent être conditionnées comme des lotions en flacon avec un réducteur.

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition. Des exemples concrets, mais nullement limitatifs, illustrant l'invention, vont maintenant être donnés.

**<u>Exemples</u>** :5 exemples de formulation anhydre 1 à 5 ont été préparés: Les quantités sont exprimées en % massique par rapport au poids total de la composition.

| Phase | Ingrédients | Exemple 1 (hors invention) |
|---|---|---|
| A | PARAFFINUM LIQUIDUM | 29,4 |
| | CAPRYLIC/CAPRIC TRIGLYCERIDE (60/40) (MYRITOL 318- COGNIS) | 20,0 |
| | ETHYLHEXYL METHOXYCINNAMATE | 7,5 |
| | DROMETRIZOLE TRISILOXANE | 2,5 |
| | PARFUM | 0.5 |
| | VITAMINE E | 0.2 |
| | COLORANT | 0,004 |

(suite)

| Phase | Ingrédients | Exemple 1 (hors invention) | | |
|---|---|---|---|---|
| B | CYCLOPENTASILOXANE | qsp | | |
| **Phase** | **Ingrédients** | **Exemple 2 (hors invention)** | | |
| A | ETHYLENEDIAMINE/STEARYL DIMER DILINOLEATE COPOLYMER (UNICLEAR 100 VG - ARIZONA CHEMICAL) | 5 | | |
| | CAPRYLIC/CAPRIC TRIGLYCERIDE (60/40) (MYRITOL 318- COGNIS) | 39 | | |
| | BUTYL METHOXYDIBENZOYLMETHANE | 2 | | |
| | ETHYLHEXYL TRIAZONE | 5 | | |
| | OCTOCRYLENE | 9 | | |
| | C12-15 ALKYL BENZOATE (TEGOSOFT TN de EVONIK GOLDSCHMIDT) | 10 | | |
| B | OCTYL-2-DODECANOL | qsp | | |
| **Phase** | **Ingrédients** | **Ex 3** | **Ex 4** | **Ex 5** |
| A | ETHYLENEDIAMINE/STEARYL DIMER DILINOLEATE COPOLYMER (UNICLEAR 100 VG - ARIZONA CHEMICAL) | 6 | 6 | - |
| | ETHYLENEDIAMINE/HYDROGENA TED DIMER | - | - | 6 |
| | DILINOLEATE COPOLYMER BIS-DI-C14-18 ALKYL AMIDE | | | |
| | CAPRYLIC/CAPRIC TRIGLYCERIDE (60/40) (MYRITOL 318- COGNIS) | 33 | 33 | 33 |
| | BUTYL METHOXYDIBENZOYLMETHANE | 4 | 4 | 4 |
| | ETHYLHEXYL TRIAZONE | 3 | 3 | 3 |
| | OCTOCRYLENE | 5 | 5 | 5 |
| | ETHYLHEXYL SALICYLATE | - | 5 | - |
| | DROMETRIZOLE TRISILOXANE | 1 | 1 | 1 |
| | BIS-ETHYLHEXYLOXYPHENOL METHOXYPHENYL TRIAZINE | - | 3 | - |
| | C12-15 ALKYL BENZOATE (TEGOSOFT TN de EVONIK GOLDSCHMIDT) | 13 | 13 | 13 |
| | OCTYL-2-DODECANOL | qsp | qsp | qsp |
| | PARFUM | 0.5 | 0.5 | 0.5 |
| | COLORANT | 0.004 | 0.004 | 0.004 |
| | VITAMINE E | 0.5 | 0.5 | 0.5 |
| B | ETHANOL | 5 | 5 | 5 |

Le mode opératoire des compositions 1 à 5 est le suivant :

On prépare la phase A en mélangeant les matières premières et on chauffe à 90-95°C sous agitation jusqu'à solubilisation totale des matières premières. On refroidit à 25°C sous agitation. On incorpore ensuite la phase B à 25°C sous agitation jusqu'à parfaite homogénéisation.

On évalue pour chacune des compositions

(1) la viscosité mesurée à l'aide d'un viscosimètre Rhéomat 180 à 25°C à la vitesse de rotation de 200 RPM après 30 secondes de rotation
(2) la stabilité à 2 mois à 4, 25 et 45°C
(3) l'aspect de la composition après 24 heures
(4) le FPS in vivo sur 5 sujets selon la méthode Internationale publiée par Colipa / CTFA SA / JCIA (Mai 2006) ;
(5) le FP $UVA_{PPD}$ sur 5 sujets selon les recommandations du JCIA (version du 15/11/1995). Les résultats sont

indiqués dans les tableaux 1 et 2 suivants :

Tableau 1

| Exemples | | Ex 1 (hors invention | Ex 2 (hors invention) |
|---|---|---|---|
| Contrôles à 24 heures | | viscosité = 0,0035 Pa.s | viscosité = 0,65 Pa.s |
| | | Huile transparente liquide 1.3 NTU | Gel huileux épais qui ne coule pas 2.3 NTU |
| Stabilité après 2 mois de stockage à 4, 25 et 45°C | | Stable | Aspect hétérogène avec exsudation d'huile : présence de parties gélifiées et non gélifiées |
| Efficacité | FPS in vivo | 7,2 (5 sujets) | Non mesurable |
| | FP $UVA_{PPD}$ in vivo | 3,0 (5 sujets) | Non mesurable |

Tableau 2

| Exemples | | Ex3 (invention) | Ex 4 (invention) | Ex 5 (invention) |
|---|---|---|---|---|
| Contrôles à 24 heures | | viscosité = 0,024 Pa.s | viscosité = 0,026 Pa.s | viscosité = 0,025 Pa.s |
| | | Huile transparente liquide 13 NTU | Huile transparente liquide 5.8 NTU | Huile transparente liquide 2.6 NTU |
| Stabilité après mois de stockage à 4, 25 et 45°C | | Stable et du même ordre que les contrôles a 24h | Stable et du même ordre que les contrôles a 24h | Stable et du même ordre que les contrôles a 24h |
| Efficacité | FPS in vivo | 20,2 | 23.8 | 24,2 |
| | FP $UVA_{PPD}$ in vivo | 11,8 | >5 | 15,4 |

Ces huiles sont donc stables au stockage après 2 mois à différentes températures (4, 25, 45°C). Elles présentent l'avantage d'être vaporisables et cosmétiquement agréables. Les FPS atteints sont supérieurs à 15 contrairement aux exemples 1 et 2.

## Revendications

**1.** Composition anhydre fluide comprenant dans un milieu cosmétiquement acceptable :

a) au moins une huile hydrocarbonée **à des concentrations allant de 30 à 99,8% en poids par rapport au poids total de la composition** et
b) au moins un filtre UV organique lipophile et
c) au moins un mono-alcool linéaire en $C_1$-$C_3$ et
d) au moins un polycondensat polyamide lipophile.

**2.** Composition selon la revendication 1, où le ou les polycondensats polyamides lipophiles répondent à la formule (B) suivante :

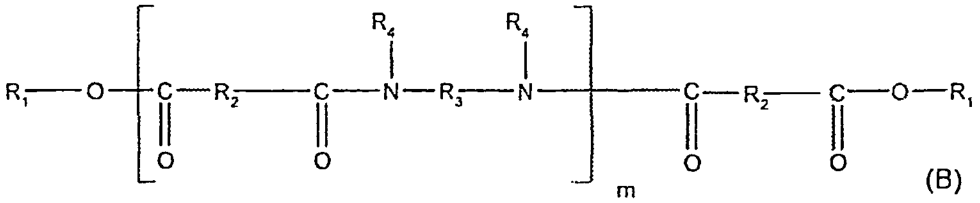

(B)

dans laquelle :

- m désigne un nombre entier de motifs amide tel que le nombre de groupes ester représente de 10 % à 50 % du nombre total des groupes ester et amide ;
- $R_1$ est à chaque occurrence indépendamment un groupe alkyle ou alcényle ayant au moins 4 atomes de carbone et notamment de 4 à 24 atomes de carbone ;
- $R_2$ représente à chaque occurrence indépendamment un groupe hydrocarboné en $C_4$ à $C_{42}$ à condition que 50 % des groupes $R_2$ représentent un groupe hydrocarboné en $C_{30}$ à $C_{42}$ ;
- $R_3$ représente à chaque occurrence indépendamment un groupe organique pourvu d'au moins 2 atomes de carbone, d'atomes d'hydrogène et optionnellement d'un ou plusieurs atomes d'oxygène ou d'azote ;
- et $R_4$ représente à chaque occurrence indépendamment un atome d'hydrogène, un groupe alkyle en $C_1$ à $C_{10}$ ou une liaison directe à $R_3$ ou à un autre $R_4$ de sorte que l'atome d'azote auquel sont liés à la fois $R_3$ et $R_4$ fasse partie d'une structure hétérocyclique définie par $R_4$-N-$R_3$, avec au moins 50 % des $R_4$ représentant un atome d'hydrogène.

**3.** Composition selon la revendication 2, où le polycondensat de formule (B) est un mélange de copolymères d'un diacide en $C_{36}$ condensé sur l'éthylène diamine ; les groupes ester terminaux résultent de l'estérification des terminaisons d'acide restantes par l'alcool cétylique, l'alcool stéarylique ou leurs mélanges (nom INCI : ETHYLENEDIAMINE/STEARYL DIMER DILINOLEATE COPOLYMER).

**4.** Composition selon la revendication 1, où le polycondensat est un copolymère de diacide linoléique hydrogéné, d'éthylènediamine et de di($C_{14}$-$C_{18}$)alkylamine(s) (nom INCI : ETHYLENEDIAMIDE/HYDROGENATED DIMER DILINOLEATE COPOLYMER BIS-DI-$C_{14}$-$C_{18}$ ALKYL AMIDE).

**5.** Composition selon la revendication 1, où le polycondensat est un copolymère de diacide linoléique hydrogéné, d'éthylènediamine, de néopentylglycol et d'alcool stéarylique (nom INCI: BIS-STEARYL ETHYLENEDIAMINE/NEOPENTYL GLYCOL/STEARYL HYDROGENATED DIMER DILINOLEATE COPOLYMER).

**6.** Composition selon l'une quelconque des revendications 1 à 5, où le ou les huiles hydrocarbonées sont présentes dans la composition de l'invention à des concentrations allant de 40 à 90% en poids par rapport au poids total de la composition.

**7.** Composition selon l'une quelconque des revendications 1 à 6, où le ou les filtres organiques lipophiles sont choisis parmi les dérivés de l'acide paraaminobenzoique, les dérivés salicyliques, les dérivés cinnamiques, les benzophénones et aminobenzophénones, les dérivés anthraniliques, les dérivés du dibenzoylméthane, les dérivés de β,β-diphénylacrylate, les dérivés du benzylidène camphre, les dérivés du phényl benzimidazole, les dérivés de benzotriazole, les dérivés triazine, les bis-résorcinyl triazines, les dérivés d'imidazolines, les dérivés du benzalmalonate, les dérivés de 4,4-diarylbutadiène, les dérivés de benzoxazole, les mérocyanines et leurs mélanges.

**8.** Composition selon l'une quelconque des revendications 1 à 7, où le mono-alcool linéaire en $C_1$-$C_3$ est l'éthanol.

**9.** Composition selon l'une quelconque des revendications 1 à 8, **caractérisée par le fait qu'**elle est transparente et présente une turbidité inférieure à 1000 NTU à 25°C, de préférence inférieure à 50 NTU à 25°C et encore plus préférentiellement inférieure à 15 NTU.

**10.** Composition selon l'une quelconque des revendications 1 à 9, **caractérisée par le fait qu**'elle présente un FPS supérieur à 10, voire supérieur à 15 et même supérieur à 20.

**11.** Composition selon l'une quelconque des revendications 1 à 10, **caractérisée par le fait qu'elle** présente un FP $UVA_{PPD}$ supérieur à 5 et/ou un ratio SPF /PPD inférieur à 3.

**Patentansprüche**

1. Wasserfreie fließfähige Zusammensetzung, umfassend in einem kosmetisch unbedenklichen Medium:

    a) mindestens ein Kohlenwasserstofföl in Konzentrationen von 30 bis 99,8 Gew.%, bezogen auf das Gesamtgewicht der Zusammensetzung, und
    b) mindestens ein lipophiles organisches UV-Filter und
    c) mindestens einen linearen $C_1$-$C_3$-Monoalkohol und
    d) mindestens ein lipophiles Polyamidpolykondensat.

2. Zusammensetzung nach Anspruch 1, wobei das oder die lipophilen Polyamidpolykondensate der folgenden Formel (B) entsprechen:

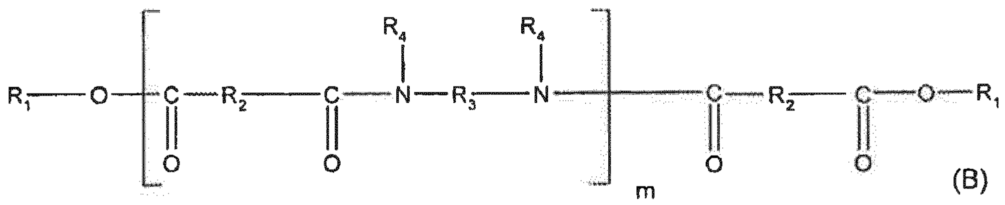
(B)

worin:

- m für eine solche ganze Zahl von Amideinheiten steht, dass die Zahl der Estergruppen 10% bis 50%, bezogen auf die Gesamtzahl der Ester- und Amidgruppen, beträgt;
- $R_1$ jeweils unabhängig für eine Alkyl- oder Alkenylgruppe mit mindestens 4 Kohlenstoffatomen und insbesondere 4 bis 24 Kohlenstoffatomen steht;
- $R_2$ jeweils unabhängig für eine $C_4$- bis $C_{42}$-Kohlenwasserstoffgruppe steht, mit der Maßgabe, dass 50% der Gruppen $R_2$ für eine $C_{30}$- bis $C_{42}$-Kohlenwasserstoffgruppe stehen;
- $R_3$ jeweils unabhängig für eine organische Gruppe mit mindestens 2 Kohlenstoffatomen, Wasserstoffatomen und gegebenenfalls einem oder mehreren Sauerstoff- oder Stickstoffatomen steht
- und $R_4$ jeweils unabhängig für ein Wasserstoffatom, eine $C_1$- bis $C_{10}$-Alkylgruppe oder eine direkte Bindung zu $R_3$ oder einem anderen $R_4$ steht, so dass das Stickstoffatom, an das sowohl $R_3$ als auch $R_4$ gebunden sind, einen Teil einer durch $R_4$-N-$R_3$ definierten heterocyclischen Struktur bildet, wobei mindestens 50% der Reste $R_4$ für ein Wasserstoffatom stehen.

3. Zusammensetzung nach Anspruch 2, wobei es sich bei dem Polykondensat der Formel (B) um ein Gemisch von Copolymeren einer mit Ethylendiamin kondensierten $C_{36}$-Disäure handelt und sich die endständigen Estergruppen aus der Veresterung der verbleibenden Säureendgruppen mit Cetylalkohol, Stearylalkohol oder Gemischen davon ergeben (INCI-Name: Ethylenediamine/Stearyl Dimer Dilinoleate Copolymer).

4. Zusammensetzung nach Anspruch 1, wobei es sich bei dem Polykondensat um ein Copolymer von hydrierter Dilinolsäure, Ethylendiamin und Di($C_{14}$-$C_{18}$)alkyl-amin(en) (INCI-Name: Ethylenediamine/Hydrogenated Dimer Dilinoleate Copolymer Bis-Di-$C_{14}$-$C_{18}$ Alkyl Amide) handelt.

5. Zusammensetzung nach Anspruch 1, wobei es sich bei dem Polykondensat um ein Copolymer von hydrierter Dilinolsäure, Ethylendiamin, Neopentylglykol und Stearylalkohol (INCI-Name: Bis-Stearyl Ethylenediamine/Neopentyl Glycol/Stearyl Hydrogenated Dimer Dilinoleate Copolymer) handelt.

6. Zusammensetzung nach einem der Ansprüche 1 bis 5, wobei das oder die Kohlenwasserstofföle in der erfindungsgemäßen Zusammensetzung in Konzentrationen von 40 bis 90 Gew.-%, bezogen auf das Gesamtgewicht der Zusammensetzung, vorliegen.

7. Zusammensetzung nach einem der Ansprüche 1 bis 6, wobei das oder die lipophilen organischen Filter aus para-Aminobenzoesäurederivaten, Salicylsäurederivaten, Zimtsäurederivaten, Benzophenonen und Aminobenzophenonen, Anthranilsäurederivaten, Dibenzoylmethanderivaten, β,β-Diphenylacrylatderivaten, Benzylidencampherderivaten, Phenylbenzimidazolderivaten, Benzotriazolderivaten, Triazinderivaten, Bisresorcinyltriazinen, Imidazolinderivaten, Benzalmalonatderivaten, 4,4-Diarylbutadienderivaten, Benzoxazolderivaten, Merocyaninderivaten und Gemischen davon ausgewählt sind.

**8.** Zusammensetzung nach einem der Ansprüche 1 bis 7, wobei es sich bei dem linearen $C_1$-$C_3$-Monoalkohol um Ethanol handelt.

**9.** Zusammensetzung nach einem der Ansprüche 1 bis 8, **dadurch gekennzeichnet, dass** sie transparent ist und eine Trübung von weniger als 1000 NTU bei 25°C, vorzugsweise weniger als 50 NTU bei 25°C und noch weiter bevorzugt weniger als 15 NTU aufweist.

**10.** Zusammensetzung nach einem der Ansprüche 1 bis 9, **dadurch gekennzeichnet, dass** sie einen LSF von mehr als 10, sogar mehr als 15 und sogar mehr als 20 aufweist.

**11.** Zusammensetzung nach einem der Ansprüche 1 bis 10, **dadurch gekennzeichnet, dass** sie einen $UVA_{PPD}$-SF von mehr als 5 und/oder ein LSF/PPD-Verhältnis von weniger als 3 aufweist.

**Claims**

**1.** Anhydrous fluid composition comprising, in a cosmetically acceptable medium:

a) at least one hydrocarbon oil at concentrations ranging from 30% to 99.8% by weight, with respect to the total weight of the composition, and
b) at least one lipophilic organic UV screening agent and
c) at least one linear $C_1$-$C_3$ monoalcohol and
d) at least one lipophilic polyamide polycondensate.

**2.** Composition according to Claim 1, where the lipophilic polyamide polycondensate or polycondensates correspond to the following formula (B):

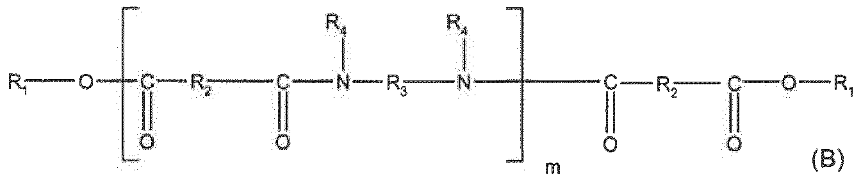

(B)

in which:

- m denotes an integral number of amide units such that the number of ester groups represents from 10% to 50% of the total number of the ester and amide groups;
- $R_1$ is in each case independently an alkyl or alkenyl group having at least 4 carbon atoms and in particular from 4 to 24 carbon atoms;
- $R_2$ independently represents, in each case, a $C_4$ to $C_{42}$ hydrocarbon group, provided that 50% of the $R_2$ groups represent a $C_{30}$ to $C_{42}$ hydrocarbon group;
- $R_3$ independently represents, in each case, an organic group provided with at least two carbon atoms, with hydrogen atoms and optionally with one or more oxygen or nitrogen atoms;
- and $R_4$ independently represents, in each case, a hydrogen atom, a $C_1$ to $C_{10}$ alkyl group or a direct bond to $R_3$ or to another $R_4$, so that the nitrogen atom to which both $R_3$ and $R_4$ are bonded forms part of a heterocyclic structure defined by $R_4$-N-$R_3$, with at least 50% of the $R_4$ groups representing a hydrogen atom.

**3.** Composition according to Claim 2, where the polycondensate of formula (B) is a blend of copolymers of a $C_{36}$ diacid condensed with ethylenediamine; the end ester groups result from the esterification of the remaining acid endings by cetyl alcohol, stearyl alcohol or their mixtures (INCI name: Ethylenediamine/Stearyl Dimer Dilinoleate Copolymer).

**4.** Composition according to Claim 1, where the polycondensate is a copolymer of hydrogenated dilinoleic acid, ethylenediamine and di($C_{14}$-$C_{18}$) alkylamine (s) (INCI name: Ethylenediamide/Hydrogenated Dimer Dilinoleate Copolymer Bis-Di-$C_{14}$-$C_{18}$ Alkyl Amide).

**5.** Composition according to Claim 1, where the polycondensate is a copolymer of hydrogenated dilinoleic acid, ethylenediamine, neopentyl glycol and stearyl alcohol (INCI name: Bis-Stearyl Ethylenediamine/Neopentyl Glycol/Stearyl Hydrogenated Dimer Dilinoleate Copolymer).

**6.** Composition according to any one of Claims 1 to 5, where the hydrocarbon oil or oils are present in the composition of the invention at concentrations ranging from 40% to 90% by weight, with respect to the total weight of the composition.

**7.** Composition according to any one of Claims 1 to 6, where the lipophilic organic screening agent or agents are chosen from para-aminobenzoic acid derivatives, salicylic derivatives, cinnamic derivatives, benzophenones or aminobenzophenones, anthranilic derivatives, dibenzoylmethane derivatives, β,β-diphenylacrylate derivatives, benzylidenecamphor derivatives, phenylbenzimidazole derivatives, benzotriazole derivatives, triazine derivatives, bisresorcinyltriazines, imidazoline derivatives, benzalmalonate derivatives, 4,4-diarylbutadiene derivatives, benzoxazole derivatives, merocyanines and their mixtures.

**8.** Composition according to any one of Claims 1 to 7, where the linear $C_1$-$C_3$ monoalcohol is ethanol.

**9.** Composition according to any one of Claims 1 to 8, **characterized in that** it is transparent and exhibits a turbidity of less than 1000 NTU at 25°C, preferably of less than 50 NTU at 25°C and more preferably still of less than 15 NTU.

**10.** Composition according to any one of Claims 1 to 9, **characterized in that** it exhibits an SPF of greater than 10, even of greater than 15 and even of greater than 20.

**11.** Composition according to any one of Claims 1 to 10, **characterized in that** it exhibits a UV-$A_{PPD}$ PF of greater than 5 and/or an SPF/PPD ratio of less than 3.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1813266 A
- EP 2014277 A
- US 5783657 A
- US 6399713 B
- US 3645705 A
- US 3148125 A
- US 5500209 A
- WO 2007068371 A
- WO 2008155059 A
- US 4077441 A
- US 4850517 A


### Littérature non-brevet citée dans la description


- Japan Cosmetic Industry Association Technical Bulletin. *Measurement Standards for UVA protection efficacy,* 21 Novembre 1995